# EUROPEAN PATENT SPECIFICATION

(11) **EP 0 317 005 B1**
(45) Date of publication and mention of the grant of the patent: **23.02.1994**
(21) Application number: 88202527.3
(22) Date of filing: 10.11.1988
(51) Int. Cl.: C07D 307/60, C07C 51/567, C07C 57/13

(54) **Process for the preparation of a substituted succinic anhydride**
Verfahren zur Herstellung von substituiertem Bernsteinsäureanhydrid
Procédé de préparation d'anhydride succinique substitué

(30) Priority: 13.11.1987 US 120257
(43) Date of publication of application: 24.05.1989
(73) Proprietor: SHELL INTERNATIONALE RESEARCH MAATSCHAPPIJ B.V., 2596 HR Den Haag (NL)
(72) Inventor: Fried, Herbert Elliott, Houston, TX 77042 (US)

(56) References cited:
- US-A- 4 086 251
- PATENT ABSTRACTS OF JAPAN, vol. 10, no. 4 (C-322)[2061], 09 January19863

## Description

This invention relates to a process for the preparation of a substituted succinic anhydride by reacting an olefinically unsaturated compound with maleic anhydride in the presence of a catalytic amount of an additive which inhibits side-reactions.

Reactions of olefinically unsaturated compounds with maleic anhydride at elevated temperatures give the corresponding adducts in accordance with the following equation: 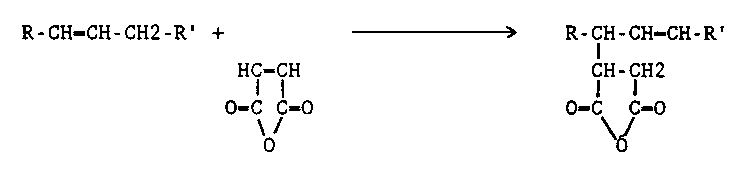
(R and R' represent independently hydrogen atoms or optionally substituted hydrocarbyl groups.)
However, reactions of this type require very long reaction times even at elevated temperatures. In addition, under conventional temperature conditions (200-300 °C), darkening of the product and formation of black solids are typically observed. These problems are believed to arise from secondary reactions involving maleic anhydride, for example, polymerization and decarboxylation. Thus, stabilization of maleic anhydride for these secondary reactions and acceleration of the addition reaction is desirable.

It is known that the reaction of the olefinically unsaturated hydrocarbons with maleic anhydride can be carried out in the presence of a catalytically effective amount of an additive in order to accelerate the reaction. For example, in conventional processes, the reaction times are reduced to a satisfactory level by carrying out the addition reactions in the presence of small amounts of substances such as furan derivatives (U.S. 4,388,471), iodine (GB 1,356,802), bromine (GB 1,480,453), an alpha-bromodialkylketone (U.S. 3,953,475 and U.S. 3,954,812), hydrogen chloride or calcium bromide (U.S. 3,935,249), a hydantoin derivative (U.S. 3,927,041), p-toluenesulfonic acid (U.S. 3,855,251), a nickel salt (GB 2,081,274) or a bromophenol (U.S. 4,278,604).

In these processes, however, the degree of conversion of the olefin is frequently low. In addition, where halogen compounds are used, extra precautions have to be taken due to the toxicity of the reaction mixture. Many of these conventional processes also have the disadvantages of producing discoloration and formation of solids during the reaction which contaminate the kettle walls or, in more adverse cases, the reaction product. An even more disadvantageous feature is the formation of resin-like residues which render the product useless if it cannot be purified by distillation or filtration.

US-A-4,086,251 discloses a process for the preparation of a substituted succinic anhydride by the reaction of a polypropene or a polybutene with maleic acid in the presence of the additive to suppress the formation of tar and side products. Examples of such additives include chlorinated or brominated aliphatic hydrocarbons, chlorine and/or bromine-containing derivatives of carboxylic or sulphonic acids, chlorinated and/or brominated intramolecular anhydrides of aliphatic carboxylic acids and several other chlorine or bromine-containing organic or inorganic compounds. It is proposed in US-A-4,599,433 to carry out the above adduction reaction in the presence of an alkoxide of titanium, zirconium, vanadium or aluminium. Titanium (IV) n-butoxide is the only alkoxide exemplified and is rather expensive.

It is known to use aluminium or compounds thereof to reduce these side-reactions. Thus it has been proposed in JP-A-57.035581 to use a relatively high amount of metallic aluminium (up to 50 %w), which has the disadvantage of poor dissolution into the reaction mixture, and requires special solvents. It is described in US 4,396,774 that alkylaluminium halides may be used in the present addition reaction, but these compounds are halogenated and necessitate the presence of halogenated solvents such as methylene chloride, which may pose environmental problems.

It is the aim of the present invention to provide a process for the preparation of substituted succinic anhydrides which can be carried out using a side-reaction inhibiting additive which does not contain halogen atoms, and which process does not necessitate the use of a solvent, so that discolouration and resin-like residue formation is avoided.

It has now been found that this objective is met by using small amounts of aluminium acetylacetonate as the additive, which reduce the formation of black solids and improve product colour for the reaction of maleic anhydride and olefinically unsaturated compounds to produce substituted succinic anhydrides. The presence of this additive permits the reaction to be conducted at higher temperatures which decreases residence times and allows complete consumption of maleic anhydride to avoid plugging or recycle.

Accordingly, the present invention relates to a process for the preparation of a substituted succinic anhydride by reacting an olefinically unsaturated compound with maleic anhydride in the presence of a catalytic amount of an additive, which inhibits side-reactions, characterized in that aluminium acetylacetonate is present as the additive.

Suitable olefinically unsaturated compounds for the process of the invention are all compounds which possess terminal double bonds or double bonds within a chain and have a molecular weight in the range of from about 100 to about 3000, and mixtures of these compounds. Though hydrocarbons (i.e. olefins) are preferred, compounds with functional groups, e.g. acrylate esters, are suitable too.

The term "olefinically unsaturated hydrocarbons" as used herein, refers to monomeric, oligomeric and polymeric C₇-C₂₀₀ alkenes whose chains may or may not be branched. The olefinic unsaturated hydrocarbons which can be subjected to the addition reaction include, for example, tetradecene-1, oct-1-ene, 2,4,4-trimethylpent-2-ene, 2-methyl-5-propylhex-1-ene, 3-cyclohexyl-bute-1-ene and the oligomers of C₂-C₂₀ olefins, for example the oligomers of ethylene, propylene, bute-1-ene, isobutene, hex-1-ene, oct-1-ene, and the like, and the polyisobutenes where the molecular weight is from 350 to 3000, and diisobutene. Preferred olefinically unsaturated hydrocarbons are C₁₄-C₂₀ linear or branched internal olefins and alpha olefins.

In the reaction of the olefinically unsaturated compounds with maleic anhydride, the molar ratio of maleic anhydride to olefin, i.e., the proportions of substances based on the number of moles of components, is typically from about 0.4:1 to about 5.0:1, preferably from 0.65:1 to 1.2:1, in particular from 0.8:1 to 1:1. A process in which equal molar amounts of olefinic compound and maleic anhydride can be used is particularly preferred.

To avoid side reactions during the addition reaction of maleic anhydride, the reaction is carried out in the presence of from 1 to 5000, preferably from 5 to 1000, ppm by weight, based on the weight of the reactants used, of aluminium acetylacetonate. The principle side reactions are believed to be the formation of poly(maleic anhydride), which is obtained as a solid residue, or poly(maleic anhydride) with an olefinic component from free radical copolymerization of the olefin and the maleic anhydride. The addition reaction with formation of the corresponding succinic anhydrides takes place at from about 160 °C to about 260 °C, preferably from 230 °C to 245 °C. The reaction is preferably carried out in an agitated reactor either in the presence or in the absence of a solvent, although no solvent is typically required. The reaction times are typically from about 1 hour to about 20 hours, preferably from 4 hours to 10 hours and especially from 3.5 hours to 5 hours. In a preferred embodiment, the reaction is carried out in an autoclave in an essentially oxygen-free atmosphere in the presence of an inert gas. A nitrogen or argon atmosphere is preferably used as the inert atmosphere. When the reaction is complete, the autoclave is left to cool and the reaction mass is preferably worked up by distillation. As far as possible, the reactants should be anhydrous.

The aluminium acetylacetonate is in the solid state and is used in this form in the addition reaction. One advantage obtained when the invention is used in the absence of solvents is the fact that less waste is produced. In addition, the use of small amounts of aluminium acetylacetonate dramatically reduces sludge-make and improves the product colour.

The resulting products having molecular weights in the range of from about 200 to about 350 are used for the preparation of anticorrosive agents for aqueous or organic systems. The resulting olefinic-succinic anhydrides having molecular weights in the range of from about 250 to about 3000 can be converted in simple manner to compounds which are suitable as oil additives such as for example, lubricant additives.

The invention will now be described by the use of the following examples. The results are presented in the Table.

### Example 1

37.0 Grams of tetradecene-1 having a molecular weight of 196 and 18.0 grams of maleic anhydride (a maleic anhydride to olefin molar ratio of 1) were reacted in a Fischer-Porter bottle in the presence of 0.01 grams of aluminium acetylacetonate while being stirred with a magnetic stirrer. The reaction mixture was then heated to 245 °C for 4 hours.

### Example 2

The procedure of Example 1 was repeated except that 0.2 grams of aluminium acetylacetonate was used as additive.

### Example 3

37.0 Grams of a C₁₅-C₂₀ internal olefin mixture having an average molecular weight of 239 was sparged with nitrogen for about 16 hours and then reacted with 12.1 grams of maleic anhydride (a maleic anhydride to olefin molar ratio of 0.8) in a Fischer-Porter bottle in the presence of 0.02 grams of aluminium acetylacetonate while being stirred with a magnetic stirrer. The reaction mixture was then heated to 230 °C for 20 hours.

### Example 4

Example 3 was repeated, except that it was carried out in the presence of 0.01 grams of aluminium acetylacetonate, and that the reaction mixture was heated to 245 °C.

### Comparative Example A

The procedure of Example 1 was repeated except that no additive was used.

### Comparative Example B

The procedure of Example 3 was repeated except that no additive was used.

### Comparative Example C

The procedure of Example 4 was repeated except that no additive was used.

**TABLE**

| Example | Reaction Time (hr) | Temp. (°C) | Olefin^{a)} (%w) | Subst.^{a)} Anhydride (%w) | Klett colour |
|---|---|---|---|---|---|
| 1 | 4 | 245 | alpha (28.7) | 71.3 | 136 |
| 2 | 4 | 245 | alpha (24.4) | 75.6 | 295 |
| 3 | 20 | 230 | internal (25.7) | 74.3 | 330 |
| 4 | 20 | 245 | internal (36.1) | 63.9 | 230 |
| A | 4 | 245 | alpha (26.5) | 73.4 | 560^{b)} |
| B | 20 | 230 | internal (38.0) | 62.0 | 286^{b)} |
| C | 20 | 245 | internal (36.2) | 63.8 | 700^{b)} |

| | | | | | |
|---|---|---|---|---|---|
| a) Determined by gas liquid chromatography. The value for substituted anhydride includes both the 1:1 and the 2:1 maleic anhydride/olefin adducts (the latter comprising less than 6%). | | | | | |
| b) Significant amounts of black solids observed. | | | | | |

As can be seen from the Table, the presence of aluminium acetylacetonate as an additive in the reaction of olefins and maleic anhydride results in a product having reduced formation of soluble and/or insoluble contaminants. In many cases, the use of this additive results in reduction of soluble by-products and improved product colour. In other cases, for instance, for that shown in Comparative Example B, the invention results in reduced formation of insoluble by-products. The Table shows that the Klett colour number for Comparative Example B is lower than that for Example 3. However, Comparative Example B, in which no additive was used, resulted in a product which contains substantial amounts of black solids and is, therefore, undesirable.

## Claims

1. Process for the preparation of a substituted succinic anhydride by reacting an olefinically unsaturated compound with maleic anhydride in the presence of a catalytic amount of an additive, which inhibits side-reactions, characterized in that aluminium acetylacetonate is present as the additive.

2. A process as claimed in claim 1, characterized in that the amount of aluminium acetylacetonate is in the range of 1 to 5000 ppm by weight, based on total weight of reactants.

3. A process as claimed in claim 2, characterized in that the amount of aluminium acetylacetonate is in the range of 5 to 1000 ppm by weight, based on total weight of reactants.

4. A process as claimed in any of claims 1-3, characterized in that an alkenyl substituted succinic anhydride is prepared by reacting a C₇₋₂₀₀ olefin with maleic anhydride.

5. A process as claimed in claim 4, characterized in that the olefin is a C₁₄-C₂₀ linear or branched internal or alpha-olefin, or a mixture thereof.

## Patentansprüche

1. Verfahren zur Herstellung eines substituierten Bernsteinsäureanhydrids durch Zur-Reaktion-bringen einer olefinisch ungesättigten Verbindung mit Maleinsäureanhydrid in Gegenwart einer katalytischen Menge eines Zusatzmittels, welches Nebenreaktionen verhindert, dadurch gekennzeichnet, daß Aluminiumacetylacetonat als Zusatzmittel vorliegt.

2. Ein Verfahren wie in Anspruch 1 beansprucht, dadurch gekennzeichnet, daß die Menge an Aluminiumacetylacetonat im Bereich von 1 bis 5000 TpM(Gew) liegt, basierend auf dem Gesamtgewicht der Reaktionsteilnehmer.

3. Ein Verfahren wie in Anspruch 2 beansprucht, dadurch gekennzeichnet, daß die Menge an Aluminiumacetylacetonat im Bereich von 5 bis 1000 TpM(Gew) liegt, basierend auf dem Gesamtgewicht der Reaktionsteilnehmer.

4. Ein Verfahren wie in irgendeinem der Ansprüche 1 bis 3 beansprucht, dadurch gekennzeichnet, daß ein alkenylsubstituiertes Bernsteinsäureanhydrid durch Zur-Reaktion-bringen eines C₇₋₂₀₀-Olefins mit Maleinsäureanhydrid hergestellt wird.

5. Ein Verfahren wie in Anspruch 4 beansprucht, dadurch gekennzeichnet, daß das Olefin ein lineares oder verzweigtes, inneres oder alpha C₁₄-C₂₀-Olefin oder ein Gemisch daraus ist.

## Revendications

1. Procédé pour la préparation d'un anhydride succinique substitué qui consiste à faire réagir un composé insaturé oléfiniquement avec de l'anhydride maléique en présence d'une quantité catalytique d'un additif, qui inhibe les réactions secondaires, caractérisé en ce que l'acétylacétonate d'aluminium est présent en tant qu'additif.

2. Procédé selon la revendication 1, caractérisé en ce que la quantité d'acétylacétonate d'aluminium est dans la gamme de 1 à 5 000 ppm en poids, par rapport au poids total de réactifs.

3. Procédé selon la revendication 2, caractérisé en ce que la quantité d'acétylacétonate d'aluminium est dans la gamme de 5 à 1 000 ppm en poids, par rapport au poids total de réactifs.

4. Procédé selon l'une quelconque des revendications 1 à 3, caractérisé en ce que l'on prépare l'anhydride succinique substitué en faisant réagir une oléfine en C₇ à C₂₀₀ avec de l'anhydride maléique.

5. Procédé selon la revendication 4, caractérisé en ce que l'oléfine est une oléfine interne ou une alpha-oléfine en C₁₄ à C₂₀, linéaire ou ramifié, ou un mélange de celles-ci.
